Europäisches Patentamt

European Patent Office

Office européen des brevets

(19)

(11) Veröffentlichungsnummer : **0 406 769 B1**

# (12) EUROPÄISCHE PATENTSCHRIFT

(45) Veröffentlichungstag der Patentschrift :
**25.08.93 Patentblatt 93/34**

(51) Int. Cl.$^5$ : **C07C 25/22, C07C 17/00**

(21) Anmeldenummer : **90112601.1**

(22) Anmeldetag : **02.07.90**

(54) **Verfahren zur Herstellung von 2-Chlor- bzw. 2-Bromnaphthalin.**

(30) Priorität : **05.07.89 DE 3922035**

(43) Veröffentlichungstag der Anmeldung :
**09.01.91 Patentblatt 91/02**

(45) Bekanntmachung des Hinweises auf die
Patenterteilung :
**25.08.93 Patentblatt 93/34**

(84) Benannte Vertragsstaaten :
**BE CH DE FR GB IT LI NL**

(56) Entgegenhaltungen :
**JOURNAL OF GENERAL CHEMISTRY USSR,
Band 24, 1954, Seiten 667-672, New York, US;
N.N. VOROZHISOV, Jr. et al.: "Catalytic conversion of halogen derivatives in the aromatic
series"**

(73) Patentinhaber : **HOECHST
AKTIENGESELLSCHAFT
D-65926 Frankfurt (DE)**

(72) Erfinder : **Neuber, Marita, Dr.
Schwanheimer Strasse 104
D-6000 Frankfurt am Main (DE)**
Erfinder : **Leupold, Ernst Ingo, Dr.
Auf der Erlenwiese 61
D-6392 Neu-Anspach (DE)**

EP 0 406 769 B1

## Beschreibung

Die vorliegende Erfindung betrifft ein verbessertes und vorteilhaftes Verfahren zur Herstellung von 2-Chlor- bzw. 2-Bromnaphthalin durch zeolith-katalysierte Isomerisierung von 1-Chlor- bzw. 1-Bromnaphthalin. Aus 2-Chlor- bzw. 2-Bromnaphthalin können Zwischenprodukte für die Herstellung von Pharmazeutika oder aromatischen Monomeren für Polyester erhalten werden.

Es ist bekannt, daß 1-Chlornaphthalin in flüssiger Phase mit Hilfe von $AlCl_3$ (L. Roux, Bull. Soc. Chim. 45, 510/521 (1886); T.L. Jacobs et. al., J. Org. Chem. 11, 27/33 (1946)) oder in der Gasphase an festen Katalysatoren wie Aluminiumoxid oder amorphem Alumosilikat (N.N. Vorozhtsov, Jr. und A.M. Beskin, J. Gen. Chem. USSR, 24, 667/672 (1954) engl. Ausgabe) zu 2-Chlornaphthalin isomerisiert werden kann. Ebenfalls beschrieben ist die Isomerisierung von 1-zu 2-Bromnaphthalin in flüssiger Phase (L. Roux, Bull. Soc. Chim. 45, 510/521 (1886), J.P. Wibaut et al., Rec. Trav. Chim. 68, 525/546 (1949)) und in der Gasphase an Silicagel (F. Meyer und R. Schiffner, Ber. 65, 67/69 (1934)) sowie an $FeCl_3$ (J.P. Wibaut et. al., Rec. Trav. Chim. 68, 525/546 (1949)).

Diese genannten Verfahren sind jedoch mit erheblichen Nachteilen behaftet. Bei Verwendung von z.B. $AlCl_3$ müssen große Mengen eingesetzt werden, damit die Isomerisierungsreaktion ablaufen kann (z.B. 1,8 mol $AlCl_3$ pro mol 1-Chlornaphthalin). Bei der Aufarbeitung wird der Katalysator zerstört, die entstehenden Salze müssen entsorgt werden. Die Bildung von teerartigen Produkten kann zwar eingeschränkt werden, wenn milde Reaktionsbedingungen gewählt werden, doch muß dann gasförmiges HCl bzw.

HBr durch die Reaktionsmischung geleitet werden. Auch bei der Isomerisierung von 1-Bromnaphthalin in der Gasphase an $FeCl_3$ auf Bimsstein wird HBr als Trägergas verwendet. Bei höheren Temperaturen zersetzt sich der Katalysator. Bei der Isomerisierung von 1-Chlornaphthalin an amorphem Alumosilikat bei 400°C desaktiviert der Katalysator sehr rasch, wenn die Reaktion ohne Trägergas oder mit $CO_2$ durchgeführt wird. Die Desaktivierung kann verlangsamt werden, wenn HCl als Trägergas verwendet wird. Für Aluminiumoxid-Katalysatoren ist HCl-Atmosphäre Voraussetzung, damit die Isomerisierungsreaktion ablaufen kann.

Es bestand somit ein Bedarf für ein verbessertes Verfahren zur Herstellung von 2-Chlor- bzw. 2-Bromnaphthalin, das sich insbesondere durch lange Katalysatorstandzeiten, durch gute Regenerierbarkeit des Katalysators sowie durch technisch einfache Durchführung in nicht korrosiver Atmosphäre auszeichnet.

Es wurde nun gefunden, daß man 2-Chlor- bzw. 2-Bromnaphthalin auf vorteilhaft Weise und unter Berücksichtigung der vorstehend genannten erwünschten Gesichtspunkte herstellen kann, indem man 1-Chlor- bzw. 1-Bromnaphthalin an einem Zeolith als Katalysator, dem in der wasserfreien und templatfreien Form die allgemeine Formel (A)

$$Z \cdot Al_2O_3 \cdot x \; SiO_2 \qquad (A)$$

zukommt, in welcher Z

$$M_2^{I}O, \; M^{II}O \; oder \; (M^{III})_2O_3 \qquad bedeutet,$$

worin $M^I$ für ein Alkalimetallatom, Ammonium oder ein Wasserstoffatom, $M^{II}$ für ein Erdalkalimetallatom und $M^{III}$ für ein seltenes Erdmetall der Ordnungszahl 57 bis 71 im Periodischen System der Elemente, bevorzugt Cer oder Lanthan, steht, und x eine Zahl von 4 bis 4000 bedeuten, mit der Maßgabe, daß falls Z für $M_2^{I}O$ steht, mindestens ein Teil der Alkalimetallatome durch Wasserstoffatome, Ammonium und/oder die genannten anderen Metallatome ausgetauscht ist, isomerisiert.

Als Katalysatoren eignen sich Zeolithe wie vorstehend definiert, deren Porenöffnungen von 10 oder 12 Tetraederatomen begrenzt werden und die durch einen Spaciousness Index (SI) > 1 charakterisiert werden. Der Spaciousness Index wird aus dem Verhältnis der Bildungsgeschwindigkeiten von n- und i-Butan beim Hydrocracken eines $C_{10}$-Naphthens an der bifunktionellen Form des Zeoliths bestimmt und ist ein Maß für die effektive Porenweite des Zeoliths (J. Weitkamp et al., Appl. Catal. 27, 207/210 (1986)). Besonders geeignet sind Zeolithe, deren Spaciousness Indices zwischen 3 und 18 liegen, wie beispielsweise die Zeolithe ZSM-12, EU-1 und Beta.

An beim erfindungsgemäßen Verfahren einsetzbaren Zeolithen seien beispielsweise folgende genannt:
Zeolith EU-1 (HEU-1 bedeutet die Protonenform dieses Zeoliths), beschrieben in EP 0 042 226;
Zeolith. ZSM-12 (HZSM-12 bedeutet die Protonenform dieses Zeoliths), beschrieben in US-PS 3 970 544;
Zeolith Beta (H Beta bedeutet die Protonenform dieses Zeoliths), beschrieben in US-PS 3 308 069;
Zeolith Y (HY bedeutet die Protonenform dieses Zeoliths), beschrieben von D.W. Breck in "Zeolite Molecular Sieves", John Wiley & Sons, Inc., New York, London, Sydney und Toronto, 1974. (Der Spaciousness Index dieses Zeolithen beträgt 21.)

Die genannten Zeolithe können nach Vorschriften aus der Literatur durch hydrothermale Synthese hergestellt werden. Nach der Kristallisation werden die Zeolithe abfiltriert, getrocknet und in oxidierender Atmosphäre, bevorzugt an Luft, calciniert, um das organische Templat aus den Poren zu entfernen. (Die templatfreie Form ist frei von Alkylammonium- oder Phosphoniumionen sowie von Aminen.)

Die Isomerisierung kann sowohl in der Gasphase als auch in der Flüssigphase durchgeführt werden, wobei die Isomerisierung in der Gasphase bevorzugt ist. Die Reaktionstemperaturen liegen zwischen etwa 200 und etwa 600°C, bevorzugt zwischen etwa 300 und etwa 400°C. Dabei sind für die Isomerisierung von 1-Bromnaphthalin etwas geringere Temperaturen als für die Isomerisierung von 1-Chlornaphthalin günstig. Der Druck ist für den Reaktionsablauf wenig entscheidend. Er kann auf Werte zwischen etwa 50 und etwa 10 000 kPa (etwa 0,5 und etwa 100 bar), bevorzugt zwischen etwa 100 und etwa 1000 kPa (etwa 1 und etwa 10 bar), eingestellt werdne. Zweckmäßigerweise arbeitet man bei Atmosphärendruck.

Bei Durchführung der Isomerisierung in der Gasphase kann der Katalysator in Form von Pellets, die ein Bindermaterial wie beispielsweise $Al_2O_3$ oder $SiO_2$ enthalten oder die binderfrei gepreßt werden, in den Reaktor eingebaut werden. 1-Chlor- bzw. 1-Bromnaphthalin kann hierbei allein oder im Gemisch mit Wasserstoff, Stickstoff oder einem anderen hinsichtlich der Reaktionsteilnehmer inerten Gas über den Katalysator geleitet werden. Vorteilhaft ist die Verwendung von Wasserstoff als Trägergas.

Die Verweilzeit des Reaktanden kann zwischen etwa 0,1 und etwa 20 s, bevorzugt zwischen etwa 1 und etwa 10 s liegen.

Eventuell vorhandene Alkalimetall-Ionen (Z in Formel (A) steht in diesem Fall für $M_2^IO$) werden anschließend durch Ionenaustausch gegen zwei- oder dreiwertige Ionen der Erdalkalimetalle oder Selten Erdmetalle oder gegen Ammonium-Ionen oder Protonen ausgetauscht. Diese saure Modifizierung ist notwendig, weil der Zeolith sonst keine katalytische Wirkung entfaltet. Dabei ist es zweckmäßig, daß mindestens 50 %, vorzugsweise mindestens 75 % der Alkalimetallionen durch die genannten anderen Ionen ausgetauscht sind. Bei 200 bis 800°C, bevorzugt bei 400 bis 550°C werden die Zeolithe durch Dehydratisierung (und Deammonisierung bei $NH_4^+$-Formen) in die katalytisch aktive Form überführt. Das Si/Al-Verhältnis der Zeolithe kann in einem weiten Bereich zwischen 2 und 2000 - je nach Zeolith-Typ - variieren. Bevorzugt sind Si/Al-Verhältnisse zwischen 10 und 150. Die Kristallitgröße kann zwischen etwa 0,01 und etwa 10 μm, bevorzugt zwischen etwa 0,1 und etwa 1 μm, liegen.

Bei einer Raumgeschwindigkeit zwischen 0,1 und 5 $h^{-1}$ (LHSV = liquid hourly space velocity) werden befriedigende Umsätze erzielt, wobei Raumgeschwindigkeiten zwischen 0,5 und 2 $h^{-1}$ besonders bevorzugt sind.

Die Isomerisierung in der Flüssigphase, die mit 1-Bromnaphthalin besser als mit 1-Chlornaphthalin verläuft, kann am einfachsten in einem Rührkessel mit suspendiertem Katalysator durchgeführt werden.

Wenn der Katalysator desaktiviert ist, kann er durch einfache Regeneration in oxidierender Atmosphäre, insbesondere Luft oder Luft/Stickstoffgemische, bei Temperaturen zwischen etwa 350 und etwa 800°C, insbesondere zwischen etwa 500 und etwa 600°C, wieder aktiviert werden.

Die Isomerisierung von 1-Chlor- bzw. 1-Bromnaphthalin kann in allen Apparaturen, die sich für Reaktionen in der Gas- oder Flüssigphase eignen, vorgenommen werden. Die Isomerisierung in der Gasphase ist technisch am einfachsten in einem Festbett-Strömungsreaktor zu handhaben. Die Reaktanden können flüssig in den Reaktor dosiert und dort vor dem Katalysatorbett verdampft werden oder vor dem Reaktor durch geeignete Maßnahmen in die Gasphase überführt und so in den Reaktor geleitet werden. Die Produkte werden nach dem Reaktor kondensiert.

Das Produktgemisch kann durch Kristallisation in die 1- und 2-Halogennaphthalinisomeren getrennt werden. Zunächst wird 2-Chlor- bzw. 2-Bromnaphthalin durch Kristallisation aus einem Lösungsmittel, wie beispielsweise Methanol oder Aceton, teilweise abgetrennt. Das an 2-Halogennaphthalin abgereicherte Gemisch beider Isomeren kann durch weitere Kristallisationsschritte getrennt werden oder aber in den Reaktor zurückgeführt und durch Isomerisierung von 1-Halogennaphthalin wieder mit dem 2-Isomeren bis maximal zur Gleichgewichtszusammensetzung angereichert werden. Bei 400°C liegen im Gleichgewicht ca. 44 % 1- und 56 % 2-Chlornaphthalin vor. Das ausgefällte 2-Halogennaphthalin kann durch ein- oder mehrmaliges Umkristallisieren weiter gereinigt werden.

Durch die erfindungsgemäß zur Anwendung gelangenden Zeolithe können hohe Umsätze an 1-Chlorbzw. 1-Bromnaphthalin bei gleichzeitig langer Katalysatorstandzeit erzielt werden. An einem amorphen Alumosilikat war trotz HCl-Atmosphäre der Umsatz an 1-Chlornaphthalin innerhalb von 4 h von 51 auf 41 % abgesunken (T = 400°C, LHSV = 0,2 $h^{-1}$, N.N. Vorozhtsov, Jr. und A. M. Beskin, J. Gen. Chem. USSR, 24, 667/672 (1954) engl. Ausgabe). Ein HEU-1 Zeolith setzte demgegenüber bei gleicher Temperatur aber 2,5-facher LHSV in Wasserstoffatmosphäre 56 % 1-Chlornaphthalin nach 1 h und 41 % nach 7 h um. Die Selektivität für die Isomerisierung von 1-Chlornaphthalin an HEU-1 ist sehr hoch und liegt bei Reaktionstemperaturen zwischen 300 und 400°C bei über 98 %. Als Nebenprodukt entsteht vor allem Naphthalin durch HCl-Abspaltung. Dichlornaphthaline werden zu weniger als 0,05 % gebildet. Bei der Umsetzung von 1-Bromnaphthalin tritt allerdings eine Transbromierung in Naphthalin und Dibromnaphthaline auf. Die Selektivität zu 2-Bromnaphthalin ist dennoch mit 95 % bei 350°C und 50%igem Umsatz sehr hoch.

3

Durch die nachstehenden Beispiele wird das erfindungsgemäße Verfahren näher erläutert, ohne darauf beschränkt zu werden.

**Beispiele**

Die katalytischen Umsetzungen wurden in einer Festbett-Strömungsapparatur durchgeführt. Der Reaktor bestand aus Quarzglas und hatte einen Innendurchmesser von 40 mm. 1-Chlor- bzw. 1-Bromnaphthalin wurde durch Spritzenpumpen flüssig zudosiert, am Reaktoreingang mit dem Trägergas vereinigt und in einer Verdampferzone vor dem Katalystorbett verdampft. Das Produktgemisch wurde nach dem Reaktor kondensiert und in bestimmten Zeitabständen gaschromatographisch analysiert. Der Katalysator wurde in Form von binderlos gepreßten Tabletten oder von Strängen mit $SiO_2$ als Binder eingesetzt. Das Katalysatorvolumen betrug 25 ml. Vor der Reaktion wurden die Zeolithe in situ bei 550°C 2 Stunden lang im Stickstoffstom aktiviert. Nach dem Versuch wurden die Zeolithe an Luft ($\dot{v}$ = 15 l/h) bei 550°C regeneriert.

In den nachstehenden Tabellen 1 und 2 sind einige Ergebnisse der katalytischen Umsetzung von 1-Chlor- bzw. 1-Bromnaphthalin an verschiedenen Zeolithen zusammengestellt.

Bei allen Versuchen betrug die LHSV 0,5 $h^{-1}$. Als Trägergas wurde Wasserstoff verwendet ($\dot{v}$ = 8 l/h, Normbedingungen).

Für die Isomerisierung von Chlornaphthalin wurde ein Gemisch eingesetzt, das 91,8 % 1-Chlornaphthalin (1-Cl-Np), 8,0 % 2-Chlornaphthalin (2-Cl-Np) und 0,2 % Naphthalin (Np) enthielt.

Das eingesetzte 1-Bromnaphthalin (1-Br-Np) hatte eine Reinheit von 98 %.

Tabelle 1   (Np bedeutet Naphthalin; HEU-1 bedeutet die Protonenform des Zeoliths
EU-1, HZSM-12 die Protonenform des Zeoliths ZSM-12,  H Beta die Protonenform
des Zeoliths Beta und HY die Protonenform des Zeoliths Y)

| Beispiel | 1 | | 2 | | 3 | | 4 | | 5 | | 6 | |
|---|---|---|---|---|---|---|---|---|---|---|---|---|
| Zeolith | HEU-1 Tabletten | | HEU-1 Tabletten | | HEU-1 Tabletten | | HZSM-12 Stränge, 21% $SiO_2$ | | H Beta Stränge, 21% $SiO_2$ | | HY Tabletten | |
| Temp., °C | 400 | | 300 | | 450 | | 400 | | 400 | | 400 | |
| Zeit, h | 1 | 7 | 1 | 7 | 1 | 7 | 1 | 7 | 1 | 7 | 1 | 2 |
| Produktzusammensetzung, % | | | | | | | | | | | | |
| Np | 1,1 | 0,5 | 0,6 | 0,3 | 3,1 | 2,3 | 0,8 | 0,5 | 3,8 | 1,2 | 7,1 | 3,6 |
| 1-Cl-Np | 44,0 | 65,5 | 47,4 | 74,3 | 42,9 | 56,5 | 45,3 | 60,8 | 41,9 | 73,5 | 80,5 | 86,3 |
| 2-Cl-Np | 54,9 | 34,0 | 52,0 | 25,4 | 54,0 | 41,2 | 53,9 | 38,7 | 54,3 | 25,3 | 12,4 | 10,1 |
| $\frac{2\text{-Cl-Np}}{\text{Cl-Np}}$ % | 55,5 | 34,2 | 52,2 | 25,5 | 55,7 | 42,2 | 54,3 | 38,9 | 56,4 | 25,6 | 13,3 | 10,5 |

EP 0 406 769 B1

## Tabelle 2

| Beispiel | 7 | | 8 | | 9 | |
|---|---|---|---|---|---|---|
| Zeolith | HEU-1 Tabletten | | HEU-1 Tabletten | | HY Tabletten | |
| Temp., °C | 250 | | 350 | | 350 | |
| Zeit, h | 1 | 3 | 1 | 3 | 1 | 2 |
| Produkt- zusammen- setzung, % | | | | | | |
| Np | 5,8 | 1,4 | 5,5 | 1,6 | 15,2 | 3,3 |
| 1-Br-Np | 56,2 | 70,0 | 41,8 | 52,1 | 55,3 | 86,9 |
| 2-Br-Np | 37,8 | 28,4 | 51,8 | 45,6 | 28,5 | 9,2 |
| DBr-Np | 0,2 | 0,2 | 0,9 | 0,7 | 1,0 | 0,6 |

DBr-Np bedeutet Dibromnaphthaline

**Beispiel 10**

20 g 1-Bromnaphthalin wurden in einem 50 ml Zweihalskolben mit Thermometer und Rückflußkühler unter Rühren mit 0,5 g pulverförmigem HEU-1-Zeolith 7 h auf 270°C erhitzt. Die Zusammensetzung des erhaltenen Produkts betrug: 0,2 % Naphthalin, 84,9 % 1-Bromnaphthalin, 14,7 % 2-Bromnaphthalin und 0,2 % Dibromnaphthaline.

Verwendet man anstelle des HEU-1-Zeoliths einen NaHEU-1-Zeolith (mit 75 % H und 25 % Na), CaEU-1-Zeolith oder LaEU-1-Zeolith und arbeitet im übrigen wie im Beispiel 10 beschrieben, so gelangt man zu einem ähnlichen Ergebnis.

**Patentansprüche**

1. Verfahren zur Herstellung von 2-Chlor- bzw. 2-Bromnaphthalin, dadurch gekennzeichnet, daß man 1-Chlor- oder 1-Bromnaphthalin an einem Zeolith als Katalysator, dem in der wasserfreien und templatfreien Form die allgemeine Formel (A)

$$Z \cdot Al_2O_3 \cdot x \, SiO_2 \qquad (A)$$

zukommt, in welcher Z

$M_2^IO$, $M^{II}O$ oder $(M^{III})_2O_3$       bedeutet,

worin $M^I$ für ein Alkalimetallatom oder Ammonium oder ein Wasserstoffatom, $M^{II}$ für ein Erdalkalimetallatom und $M^{III}$ für ein seltenes Erdmetall der Ordnungszahl 57 bis 71 im Periodischen System der Elemente steht, und x eine Zahl von 4 bis 4000 bedeuten, mit der Maßgabe, daß falls Z für $M_2^IO$ steht, mindestens ein Teil der Alkalimetallatome durch Wasserstoffatome, Ammonium und/oder die genannten anderen Metallatome ausgetauscht sind, isomerisiert.

6

**2.** Verfahren nach Anspruch 1, dadurch gekennzeichnet, daß die effektive Porenweite des Zeoliths durch einen Spaciousness Index > 1 charakterisiert ist.

**3.** Verfahren nach mindestens einem der Ansprüche 1 und 2, dadurch gekennzeichnet, daß die effektive Porenweite des Zeoliths durch einen Spaciousness Index von etwa 3 bis etwa 18 charakterisiert ist.

**4.** Verfahren nach mindestens einem der Ansprüche 1 bis 3, dadurch gekennzeichnet, daß die Isomerisierung in der Gasphase vorgenommen wird.

**5.** Verfahren nach mindestens einem der Ansprüche 1 bis 3, dadurch gekennzeichnet, daß die Isomerisierung in der Flüssigphase vorgenommen wird.

**6.** Verfahren nach mindestens einem der Ansprüche 1 bis 5, dadurch gekennzeichnet, daß die Isomerisierung bei Temperaturen von etwa 200 bis etwa 600°C vorgenommen wird.

**7.** Verfahren nach mindestens einem der Ansprüche 1 bis 6, dadurch gekennzeichnet, daß die Isomerisierung bei Temperaturen von etwa 300 bis etwa 400°C vorgenommen wird.

**8.** Verfahren nach mindestens einem der Ansprüche 1 bis 7, dadurch gekennzeichnet, daß die Isomerisierung bei Atmosphärendruck vorgenommen wird.

**9.** Verfahren nach mindestens einem der Ansprüche 1 bis 8, dadurch gekennzeichnet, daß die Isomerisierung in einem Druckbereich von etwa 50 bis etwa 10 000 kPa vorgenommen wird.

**10.** Verfahren nach mindestens einem der Ansprüche 1 bis 7 und 9, dadurch gekennzeichnet, daß die Isomerisierung in einem Druckbereich von etwa 100 bis etwa 1000 kPa vorgenommen wird.

**11.** Verfahren nach mindestens einem der Ansprüche 1 bis 4 und 6 bis 10, dadurch gekennzeichnet, daß man das 1-Chlor- oder 1-Bromnaphthalin in Abwesenheit eines gegenüber den Reaktionsteilnehmern inerten Gases über den Zeolith-Katalysator leitet.

**12.** Verfahren nach mindestens einem der Ansprüche 1 bis 4 und 6 bis 10, dadurch gekennzeichnet, daß man das 1-Chlor- oder 1-Bromnaphthalin im Gemisch mit einem gegenüber den Reaktionsteilnehmern inerten Gas über den Zeolith-Katalysator leitet.

**13.** Verfahren nach mindestens einem der Ansprüche 1 bis 4 und 6 bis 12, dadurch gekennzeichnet, daß man das 1-Chlor- oder 1-Bromnaphthalin im Gemisch mit Wasserstoff oder Stickstoff über den Zeolith-Katalysator leitet.

## Claims

**1.** A process for the preparation of 2-chloro- or 2-bromonaphthalene, which comprises isomerizing 1-chloro- or 1-bromonaphthalene over a zeolite as a catalyst which, in the anhydrous and template-free form, has the general formula (A)

$$Z \cdot Al_2O_3 \cdot x \, SiO_2 \qquad (A)$$

in which Z denotes

$M_2^IO$, $M^{II}O$ or $(M^{III})_2O_3$,

in which $M^I$ represents an alkali metal atom or ammonium or a hydrogen atom, $M^{II}$ represents an alkaline earth metal atom and $M^{III}$ represents a rare earth metal of atomic number 57 to 71 in the Periodic Table of the Elements, and x denotes a number from 4 to 4,000, with the proviso that if Z represents $M_2^IO$, at least some of the alkali metal atoms are replaced by hydrogen atoms, ammonium and/or the other metal atoms mentioned.

**2.** The process as claimed in claim 1, wherein the effective pore width of the zeolite has a spaciousness index > 1.

**3.** The process as claimed in at least one of claims 1 and 2, wherein the effective pore width of the zeolite has a spaciousness index of about 3 to about 18.

4. The process as claimed in at least one of claims 1 to 3, wherein the isomerization is carried out in the gas phase.

5. The process as claimed in at least one of claims 1 to 3, wherein the isomerization is carried out in the liquid phase.

6. The process as claimed in at least one of claims 1 to 5, wherein the isomerization is carried out at temperatures of about 200 to about 600°C.

7. The process as claimed in at least one of claims 1 to 6, wherein the isomerization is carried out at temperatures of about 300 to about 400°C.

8. The process as claimed in at least one of claims 1 to 7, wherein the isomerization is carried out under atmospheric pressure.

9. The process as claimed in at least one of claims 1 to 8, wherein the isomerization is carried out under a pressure in the range of about 50 to about 10,000 kPa.

10. The process as claimed in at least one of claims 1 to 7 and 9, wherein the isomerization is carried out under a pressure in the range of about 100 to about 1000 kPa.

11. The process as claimed in at least one of claims 1 to 4 and 6 to 10, wherein the 1-chloro- or 1-bromonaphthalene is passed over the zeolite catalyst in the absence of a gas which is inert towards the reactants.

12. The process as claimed in at least one of claims 1 to 4 and 6 to 10, wherein the 1-chloro- or 1-bromonaphthalene is passed over the zeolite catalyst as a mixture with a gas which is inert towards the reactants.

13. The process as claimed in at least one of claims 1 to 4 and 6 to 12, wherein 1-chloro- or 1-bromonaphthalene is passed over the zeolite catalyst as a mixture with hydrogen or nitrogen.


**Revendications**

1. Procédé de préparation du 2-chloro- ou du 2-bromo-naphtalène, caractérisé en ce qu'on soumet du 1-chloro - ou du 1-bromo-naphtalène à isomérisation sur une zéolite comme catalyseur, laquelle a, sous forme anhydre et sans gabarit, la formule générale (A) :
$$Z . Al_2O_3 . x\ SiO_2 \qquad (A)$$
dans laquelle Z représente
$M_2^IO$, $M^{II}O$ ou $(M^{III})_2O$
(formules dans lesquelles $M^I$ représente un atome de métal alcalin ou un groupe ammonium, ou un atome d'hydrogène; $M^{II}$ représente un atome de métal alcalino terreux et $M^{III}$ représente un métal des terres rares ayant pour numéro d'ordre 57 à 71 dans le Système ou Tableau Périodique des Eléments; et x est un nombre valant 4 à 4000, à la condition que si Z représente $M_2^IO$ , au moins une partie des atomes de métaux alcalins sont remplacés par échange par des atomes d'hydrogène, par un groupe ammonium et/ou les autres atomes de métaux cités).

2. Procédé selon la revendication 1, caractérisé en ce que la largeur efficace des pores de la zéolite se caractérise par un "Spaciousness Index" >1 .

3. Procédé selon l'une au moins des revendications 1 et 2, caractérisé en ce que la largeur efficace des pores de la zéolite se caractérise par un "Spaciousness Index" d'environ 3 à environ 18.

4. Procédé selon l'une au moins des revendications 1 à 3, caractérisé en ce que l'isomérisation est entreprise en phase gazeuse.

5. Procédé selon l'une au moins des revendications 1 à 3, caractérisé en ce que l'isomérisation est entreprise en phase liquide,

6. Procédé selon l'une au moins des revendications 1 à 5, caractérisé en ce que l'isomérisation est réalisée à des températures d'environ 200 à environ 600°C.

7. Procédé selon l'une au moins des revendications 1 à 6, caractérisé en ce que l'isomérisation est réalisée à des températures d'environ 300 à environ 400°C.

8. Procédé selon l'une au moins des revendications 1 à 7, caractérisé en ce que l'isomérisation est réalisée sous la pression atmosphérique

9. Procédé selon l'une au moins des revendications 1 à 8, caractérisé en ce que l'isomérisation est réalisée dans un domaine de la pression d'environ 50 à environ 10 000 kPa.

10. Procédé selon l'une au moins des revendications 1 à 7 et 9, caractérisé en ce que l'isomérisation est réalisée dans un domaine de la pression d'environ 100 à environ 1000 kPa.

11. Procédé selon l'une au moins des revendications 1 à 4 et 6 à 10, caractérisé en ce qu'on fait passer sur le catalyseur zéolitique le 1-chloro-naphtalène ou le 1-bromo-naphtalène en l'absence d'un gaz inerte à l'égard des corps participant à la réaction.

12. Procédé selon l'une au moins des revendications 1 à 4, et 6 à 10, caractérisé en ce qu'on fait passer sur le catalyseur zéolitique le 1-chloronaphtalène ou le 1-bromo-naphtalène en mélange avec un gaz inerte à l'égard des corps participant à la réaction.

13. Procédé selon l'une au moins des revendications 1 à 4 et 6 à 12, caractérisé en ce qu'on fait passer sur le catalyseur zéolitique le 1-chloro-naphtalène ou le 1-bromo-naphtalène en mélange avec de l'hydrogène ou avec de l'azote.